# EUROPEAN PATENT SPECIFICATION

(11) **EP 1 286 696 B1**
(45) Date of publication and mention of the grant of the patent: **15.11.2006**
(21) Application number: 01938851.1
(22) Date of filing: 05.06.2001
(51) Int. Cl.: A61K 39/095, C12M 1/00

(54) **OUTER MEMBRANE VESICLE VACCINE AGAINST DISEASE CAUSED BY NEISSERIA MENINGITIDIS SEROGROUP A AND PROCESS FOR THE PRODUCTION THEREOF**
IMPFSTOFF UMFASSEND AUSSENMEMBRANVESIKEL GEGEN KRANKHEITEN VERURSACHT DURCH I NEISSERIA MENINGITIDIS/I SEROGRUPPE A UND VERFAHREN ZUR HERSTELLUNG DESSELBEN
VACCIN COMPRENANT DES VESICULES DE MEMBRANE EXTERNE UTILISE CONTRE LES MALADIES CAUSEES PAR LE SEROGROUPE A DE I NEISSERIA MENINGITIDIS /I ET METHODE DE PREPARATION

(30) Priority: 02.06.2000 NO 20002828
(43) Date of publication of application: 05.03.2003
(73) Proprietor: Statens Institutt for Folkehelse, 0403 Oslo (NO)
(72) Inventor: ROSENQVIST, Einar, National Inst of Public Health, N-0403 Oslo (NO); HARBAK, Kari, National Inst of Public Health, N-0403 Oslo (NO); NORD, Karin, c/o National Inst of Public Health, N-0403 Oslo (NO); NOSS, Lisbeth Meyer, c/o Nat Inst of Public Health, N-0403 Oslo (NO); AASE, Audun, National Institute of Public Health, N-0403 Oslo (NO)
(74) Representative: Marsden, John Christopher
(86) International application number: PCT/NO2001/000232
(87) International publication number: WO 2001/091788

(56) References cited:
- WO-A-02/09643
- WO-A1-00/48703
- WO-A1-92/16232
- WO-A2-01/09350
- US-A- 5 324 442
- FREDRIKSEN J.H. ET AL.: 'Production, characterization and control of MenB-vaccine 'Folkehelsa': An outer membrane vesicle vaccine against group B meningococcal disease' NIPH ANNALS vol. 14, no. 2, December 1991, pages 67 - 80, XP002948832
- IVO CLAASSEN ET AL.: 'Production, characterization and control of a Neisseria meningitidis hexavalent class 1 outer membrane protein containing vesicle vaccine' VACCINE vol. 14, no. 10, 1996, pages 1001 - 1008, XP002948833

## Description

The present invention concerns a proteinaceous vaccine against the causative agent for bacterial meningitis, i.e. *Neisseria meningitidis,* as well as a process for producing such a vaccine.

### Background for the invention

*N. meningitidis* is one of the most common causes of purulent meningitis all over the world. Large epidemics caused by meningococci have spread during the last decade throughout vast areas of Africa in the region referred to as the "Meningitis Belt". Globally, this organism causes each year about 300,000 cases and about 30,000 deaths, and most of these are children.

Meningococci are commonly classified into serogroups, serotypes, serosubtypes and immunotypes. This classification is based on differences between the strains related to their antigenic capsular polysaccharides, outer membrane proteins, and lipopolysaccharides.

Based on antigenic properties of capsular polysaccharides, meningococci have been divided into the 12 "serogroups" A, B, C, 29E, H, I, K, L, W-135, X, Y and Z; the serogrouping usually being done by agglutination with monoclonal antibodies or polyclonal sera.

The meningococci are subclassified according to the antigenic components of their outer membrane, and there exist large antigenic differences among strains. The meningococci are accordingly classified into more than 20 "serotypes" by using monoclonal antibodies directed towards specific conformational epitopes on the PorB protein molecule. Strains are further divided into a high number of "serosubtypes" based on differences in the PorA protein molecule.

The "immunotype" of meningococci relates to the type of lipopolysaccharide (LPS) occurring in the outer membrane. Generally, on account of the phase variation, several LPS epitopes or incomplete epitopes may co-exist on one organism so that the immunotype differs to such a degree that it does not give any reliable and clear-cut identification for classification purposes. Hetereogeneity in the assembly of the oligosaccharide in the core part also contributes to structural and antigenic variations.

On account of the large variations in the epitopes existing on the meningococcal surface, it is not at all obvious that a given vaccine against one serogroup or serotype of *N*. *meningitidis* is active against another serogroup or serotype of the bacterium, and there may even exist variations within one and the same serogroup/type making the situation quite unpredictable.

About 90% of epidemic cases of systemic meningococcal disease are caused by *N. meningitidis* of the serogroups A, B or C. Serogroup A is the cause of the majority of the cases both in endemic and epidemic situations, including the explosive epidemic outbreaks in the "Meningitis Belt" in Africa. Meningitis epidemics caused by serogroup B have occurred in Europe, Latin-America, USA and New Zealand, and serogroup C meningococci have recently caused several outbreaks in Europe, North America and Austral-Asia where especially newborns and young adults are attacked.

Concerning the epidemics in the "Meningitis Belt" in Africa caused by *N. meningitidis,* such epidemics are almost always caused by meningococci of serogroup A. Immunisation with safe and effective vaccines is believed to be the most efficient way of combating epidemics in this region.

Vaccines directed against various serogroups of meningococci have been elaborated to provoke antibodies against the corresponding serogroup polysaccharides. Thus, efficient polysaccharide vaccines exist to the common serogroups A and C meningococci, as well as to the less common serogroups W135 and Y.

In contrast, attempts at making polysaccharide vaccines against the serogroup B meningococci have failed, as they proved inefficient to produce antibodies, and thus other surface antigens have been investigated for their immunogenic properties, such as outer membrane proteins or lipopolysaccharides.

Recently, some experimental work has been reported on serogroup B meningococcal vaccines based on meningococcal outer membrane proteins (Fredriksen J.H. et al (NIPH Annals, 14(2), (1991), 67-80), Claassen, I. et al., Vaccine, 14(10), (1996), 1001-1008).

All currently available vaccines against disease caused by meningococci of serogroup A are saccharide based vaccines (see for example WO/92 16232).

Such vaccines, however, appear not to induce any long-term immunological memory, and they do not provide adequate protection for children below two years of age, as is typical of all polysaccharide-based vaccines.

There is thus still an urgent need for a vaccine against serogroup A disease that induces long-term immunological memory in all age groups, to be included in vaccination programs. It has been realised that protein may be an essential component to confer the wanted long-term immunological effects, and this has led to protein-polysaccharide conjugate vaccines currently being under investigation.

However, in contrast to what has been found for serogroup C, protein-polysaccharide conjugate vaccines in development against serogroup A meningococcal disease have not yielded the positive results hoped for. There exists thus a need for a safe and effective vaccine against meningitis caused by *N. meningitidis* serogroup A, preferably comprising a protein component.

A protein-based meningococcal serogroup A vaccine based on a serotype 4 strain has been described by L.Y.Wang and coworkers (Proceedings of the Ninth International Pathogenic Neisseria Conference (1994) p. 435-436; Pathobiology and Immunobiology of *Neisseriaceae* (1994) 940-946). Their vaccine was based on outer membrane proteins extracted by salt, and further precipitated using ethanol, and it was highlighted that no detergents were used. Such ethanol treatment render highly denaturing conditions, and is highly likely to present the proteins in a denatured state. Contents of LPS in the vaccine seems to have been surprisingly high.

### General disclosure of the invention

The surface structures of the meningococci are decisive for adhesion, colonisation, invasion and pathogenesis. Several of these structures are extremely phase variable, and the organism is thereby in a better position to avoid being detected by the host's immune defences.

In similarity with other Gram-negative bacteria, the cell wall has an inner cytoplasmic membrane, a thin intermediate peptidoglycan layer and an outer membrane (OM). The OM comprises *inter alia* outer membrane proteins, e.g. porins, phospholipids, and LPS. As an external loosely bound layer may be found the capsular polysaccharides. All these components may in principle form the basis for vaccines, provided they are through the preparatory steps kept in an immunogenic conformation, also exposed in the final vaccine product.

However, finding a method for providing such immunogenic and properly presenting forms of the relevant components of the outer membrane is not at all obvious for the person skilled in the art, and any amount of experimenting may not lead to success due to e.g. difficulties in isolation and purification, avoiding conformational changes in the relevant antigenic epitopes and denaturation of the product during isolation/purification, further minimising the forming of aggregates or agglomerates which may complicate the isolation and purification. These difficulties, which may essentially relate to the isolation and purification procedure, come in addition to difficulties in obtaining a material which is suitable to be included in a vaccine, i.e. which is immunogenic and presents the relevant antigenic epitopes to the immune system in a way that will elicit antibodies against such epitopes and which additionally will confer a long-time immunological memory effect against such epitopes.

As indicated above, vaccines based on the polysaccharide component of *N*. *meningitidis* serogroup A have not been sufficiently effective for providing long term protection against meningitis caused by serogroup A infections, and this is especially true for infants. For this reason, it has been our intention to develop a protein-containing vaccine directed against meningococcal serogroup A disease.

The proteins in the outer membrane of the bacterium are important both for stabilising the outer membrane structure and for controlling the transport through the membrane. Between 50 and 100 different outer membrane proteins are presently known, some of these being present in large proportions and called "major outer membrane proteins" (MOMPs: PorA, PorB, Rmp, Opa and Opc; the two latter are class 5-proteins). These proteins are also of significant importance for the efficiency of a protein-based vaccine.

By a number of techniques, the outer membrane of *N*. *meningitidis* may be disintegrated to further reform as outer membrane vesicles (OMV) at the order of 1/10 the size of the bacterium. These vesicles may then include most components of the original outer cell membrane, in proportions depending on techniques employed for their manufacture. Stability, size and immunogenicity may similarly depend on process parameters.

Although a vaccine based on OMV of *N. meningitidis* serogroup B is known and has been shown to be efficient in vaccine trials in Norway and Cuba, such a vaccine was less effective in Chile, where the epidemics are caused by other serotypes than the serotype used in preparing the B vaccine.

Consequently, the success of a vaccine based on antigens from one bacterium may not prove efficient as a vaccine against a bacterium of another serotype.

It was thus very surprising that when tested in mice, a vaccine prepared according to the invention, based on outer membrane vesicles from *N. meningitidis* serogroup A, gave a strong bactericidal activity against all meningococcal strains collected from several African countries.

Generally, to obtain the vaccine, a representative strain of *N. meningitidis* serogroup A is grown in a suitable growth medium being obvious to the person skilled in the art; the bacterial mass is then extracted with a mild detergent, preferably a bile acid salt, for example such as deoxycholate (DOC). DOC has several advantageous effects such as efficient killing the bacteria, disaggregating the OM to form the desired OM vesicles while presenting the OM proteins in a native state, dissolving and releasing most of the toxic lipopolysaccharides from the outer membrane, and also making the LPS remaining in the vaccine less toxic.

In such a procedure OMVs are created spontaneously, and they are purified by a suitable method, e.g. by high speed centrifugations, and filtrations.

The procedure gives an OMV preparation useful as a vaccine to be administered, e.g. by injection, directly into the individual for immunisation against serogroup A meningococcal infection. However, it is highly preferably that OMV is first combined with carriers or adjuvants such as Al(OH)₃. The vaccine may additionally comprise other vaccine components against meningitis group A, for example based on a polysaccharide or lipopolysaccharide component of the bacterium.

### Choice of bacterial strains for OMV vaccine production.

Due to the strong variation in meningococcal proteins, i.e. serotype and serosubtype patterns, an important preparatory step is to collect strains from the epidemic area and classify them by a number of specific antibodies, to have an overall view of patterns and frequences of serotypes, serosubtypes, immunotypes, etc. Provided there is a dominating clone causing the epidemic, this will strengthen the basis for a vaccine production. Such mapping of protein patterns of epidemic strains is rather indispensable, in contrast to the situation when a polysaccharide vaccine is to be used.

It has been shown that the African epidemics or pandemics have been caused by a single clone for several years, affecting a number of neighbouring countries.

Several meningococcal strains of serogroup A were collected from isolated material from different parts of the world (see Table 1), and the following procedure was performed to assist in choosing representative serogroup A strains of the bacterium to provide relevant antigenic material for use in a vaccine according to the present invention.

Meningococcal strains were investigated by the DOT-BLOT-method for characterising the surface antigens of the bacteria using specific monoclonal antibodies. In this procedure inactivated bacterial cells are fixed directly on nitrocellulose paper strips which then were reacted with solutions containing separate monoclonal antibodies, and enzyme-linked IgG directed towards the monoclonal antibodies are then added. By providing a specific substrate for the relevant enzyme, the enzyme will transform this substrate so that bacteria with the antigens bound to the antibodies are covered with a red, insoluble product.

All serogroup A strains collected from Africa expressed the PorB serotypes 4 and 21 and the PorA subtypes P1.9 and P1.20. The *N. meningitidis* strains MK83/94 (from Mali) and MK100/97 (from Togo) were chosen as representative serogroup A strains from clone III-1 in Africa, assumed to be useful for producing vaccine materials according to the present invention.

The proteins being present on the surface of relevant bacteria, and forming the basis for the proteinaceous vaccine according to the present invention, were for these strains PorB, PorA, Rmp and the Class 5-protein Opc.

A meningococcal serogroup A OMV vaccine according to the invention may be prepared by any process that is capable of extracting and releasing outer membrane proteins (OMP) and associated surface antigens, and subsequently presenting the released proteins exposed on the surface of OM vesicles in an immunogenic and essentially native conformation.

One preferred embodiment of a process for preparation of a vaccine according to the invention is generally to cultivate the selected meningococcal strain in a fermentor, followed by extraction of membrane proteins with a mild bile acid salt detergent so as to form OM vesicles, and further isolating and purifying the desired OMV as described in more detail below.

In order to preserve the native conformation of proteins and other labile OM antigens, only mild conditions should be used for preparation of OMV. Thus, ordinary heat inactivation of bacteria, e.g. at 56 °C, is preferentially avoided, as should solvent denaturation. The consequence is that during growth of bacteria and the initial steps thereafter, highly infectious material is present, involving a risk for the laboratory personnel.

Cultivation in a fermentor of a meningococcal strain, selected to be representative of or crossreacting with strains of the target epidemic, may be done in any cultivation medium known to be suitable for meningococci, e.g. in the semisynthetic Frantz' medium, or the fully synthetic modified Catlin's medium C6. Cultivation is advantageously done until early stationary phase under conditions well known in the art, monitoring parameters such as dissolved oxygen, gas flow, pH and OD. The pH is preferred initially to be about 7, and may be allowed to decrease under growth, preferably by no more than 1 pH unit and maximally by 2 units. Cultivation is terminated after end of logaritmic growth at about 6-8 h, when OD usually is above 4, preferably as high as 5 to 8.

Concentration of the bacterial cell suspension after culturing is advantageous. Though such concentration may be done in several ways, simple centrifugation of the infectious bacterial cells should preferably be avoided, so as to eliminate formation of infectious aerosol. Thus, the suspension may preferably be concentrated in a closed filtration system, for example as part of an integrated system as outlined above, with a closed loop causing a tangential flow over a membrane filter ("cross flow filtration"; CFF). By keeping the transmembrane pressure low during most of the concentration step, e.g. below about 0.5 bar, clogging of the filter with accompanying reduction in liquid flow and yield can be minimised. Using a commercial CFF system, we have long experience that a bacterial cell suspension can be transferred by pumping in closed tubings from a fermentor, and concentrated under strictly secure conditions by a factor of some 5 to 20 times, preferably 10 to 15 times, in a short period of time. As an example, 50 liter of bacterial culture can be concentrated to about 5 liter in 3/4 to 1 hour.

During the concentration process it is advantageous to carefully monitoring a number of parameters, in order to decide when concentration should be terminated. Useful parameters may be liquid flow and/or flow rate; remaining volume or weight of concentrate in the tank, for instance using weighing cells; OD; and pressures, especially such as transmembrane pressure. Monitoring of one or more of these parameters will secure that the consistency and viscosity of the concentrated suspension is appropriate for the subsequent purification steps. For each bacterial strain and cultivation medium an inexcessive amount of work may be required to establish the preferred concentration factor and consistency of the concentrate; a too heavily concentrated suspension may form aggregation and clumping of the cells.

A high transmembrane pressure, e.g. above 1 bar, or a rapid increase of this pressure, are according to our experience useful indications for the concentration process step to be terminated.

Inactivation of viable bacteria may preferably be done by addition of a mild detergent to secure that the native protein conformation is preserved. The detergent may e.g. be a bile acid salt such as deoxycholate (DOC), whereas denaturating agents such as alcohols may deform the proteins into conformations without the desired immunogenicity, and hence should be avoided. In the integrated system according to the invention, inactivation is performed upon addition of detergent to the tank being part of the CFF unit.

In previous meningococcal vaccines, including a meningococcal group B vaccine produced by us, a mercury compound (e.g. sodium thiomersal) was incorporated to secure sterility. The process according to the invention is regarded safe, both from an infectious risk perspective, and with regard to excluded possibilities of contamination of the vaccine. This confidence is for a large part based on the closed integrated system comprising cultivation, concentration and inactivation/extraction, as well as the possibility of having a final sterile filtration of the purified OMV preparation. We have now established that it is possible to dispense of the mercury compound, which further add to the good tolerance of the serogroup B vaccine when injected in humans.

Identical concentrations of the detergent are expected to effect complete killing of meningococci and lysis of the OM. For security reasons, the minimum concentrations and times for lysis should be determined for a given bacterium or strain. In practice we use 0.5% DOC for 30 min, but have found that as little as 0.16 % for 15 min is sufficient for complete killing, yielding extensive extraction of proteins which are located with preserved antigenicity in OMV. It is assumed that this concentration may still be lowered by a factor of 2 or 4.

### Adjustment of pH followed by mild detergent extraction.

According to the invention, OM is dissociated using a mild detergent, preferably a bile acid salt. Such detergents have a rigid ring structure, and upon micelle formation they have peculiar capabilities of interfering with phospholipid-containing membranes, e.g. as found in Gram negative bacteria. A number of bile salt detergents are known, e.g. salts of lithocholic acid, chenodeoxycholic acid, deoxycholic acid, ursodeoxycholic acid, cholic acid and ursocholic acid; the sodium salt of deoxycholic acid has proved well suited for dissociating the OM of neisseria. It is an advantage that this compound is atoxic in small amounts, owing to its endogenous character.

A number of conditions will strongly influence the physical state of bile acid salts such as sodium deoxycholate. Thus, temperature, pH and detergent concentration must be delicately balanced to obtain extensive dissociation of the OM and complete killing of viable bacteria, simultaneously retaining a native conformation of OM antigenic proteins in the final OMV product. The bile acid salt detergent should under the conditions used be capable also to substantially decreasing the level of the toxic LPS in OMV, although it is regarded an advantage that some LPS remain in the OMV, contributing to the immunogenicity, and presumably assisting in preservation of a native protein conformation. Bile acid anions being incorporated in the vesicle membrane apparently will also detoxify LPS, possibly by interacting with their toxic lipid A part.

The pH has a strong effect upon the physical state of bile acids. Thus, above about pH 8 deoxycholate molecules form aggregates of about 15 entities (D.M.Small, Adv Chem Ser 84 "Molecular Association in Biological and Related Systems" (1968) 31-51). If pH is lowered, there is a small and gradual increase in the aggregate size, and at about pH 7.5 a sharp phase transition occurs as the number of molecules in aggregates increase rapidly. At about pH 7.3 aggregates comprise 500 molecules or more, and a gelling will appear. Hence such gelling or precipitation must definitely be avoided during the extraction.

Since deoxycholic acid has a pKa of about 6.3, it is according to the invention judged important that pH in a deoxycholate-containing aqueous medium is carefully kept well above its pKa, preferably by some 2 pH units. Keeping pH as high as about 8.3, for example between about 8.1 and 8.5, would render the deoxycholate ion as the fully dominant molecular form - close to 100% - avoiding any tendencies of major aggregates to form.

Temperature is also of importance, as formation of aggregates is appreciably larger at 20 °C than at 35 °C. At lower temperatures, e.g at 4 °C, this aggregation tendency is still stronger.Bile acid salt concentration is of importance, as micelles or minor aggregates form at concentrations above the critical micelle concentration (CMC). For deoxycholate, CMC is about 2 to 5 mM (0.1 to 0.2%) at pH values above 8 (at about 25 °C).

Without being bound by theory, we assume that an efficient dissociation of the bacterial OM is facilitated by conditions where the bile acid occurs essentially completely in the ionic form, to prevent formation of major aggregates, and in a concentration sufficient to keep the bile acid molecules in micellar form. For this reason, a concentration down to about the CMC (for DOC about 0.1 to 0.2 %) may preferably be chosen.

Accordingly, we suggest to use some 0.05 to 2.5% DOC, preferably 0.25 to 0.75% and most preferred 0.5% DOC at about pH 8.1-8.4 for the extraction step. As the concentrated bacterial suspension usually has a low pH, e.g. about pH 6.5, the suspension needs to be pH-adjusted, e.g. to about pH 8.1 to 8.3 by adding a buffer, for example a pH 9 Tris-HCl buffer, before any bile salt detergent is added by stirring. In the integrated system according to the invention, the addition of e.g. a pH 9 buffer and a buffer containing e.g. 10% DOC, to give a final DOC concentration of 0.5%, can easily be performed without any contamination risk.

The exact detergent concentrations, pH and extraction times should, however, be established separately for other detergents as described above, based on data for CMC and pKa; this may be done without any excessive work.

The stability of OMV is typically also facilitated by divalent cations such as Ca⁺⁺, and hence the dissociation of OM and subsequent purification of OMV may be enhanced by including a chelator of divalent cations, e.g. EDTA.

### Integrated production system for high-risk process steps

We have now found that the previous steps of cultivation, concentration, inactivation and extraction may advantageously be performed in a closed, integrated system comprising a fermentor and a multipurpose treatment tank connected to a membrane filtration module. The tank may be used for inactivation and extraction processes. Connecting conduits/tubings, reservoir tanks, valves and pumps further contribute to a safe integrated system where all high-risk process steps involving infectious bacterial cells may be performed with minimal manual handling, and may optionally be automated.

The integrated system for production of an OMV-containing extract (shown below), is a part of the process leading to purified OMV, and represents an aspect of the present invention.

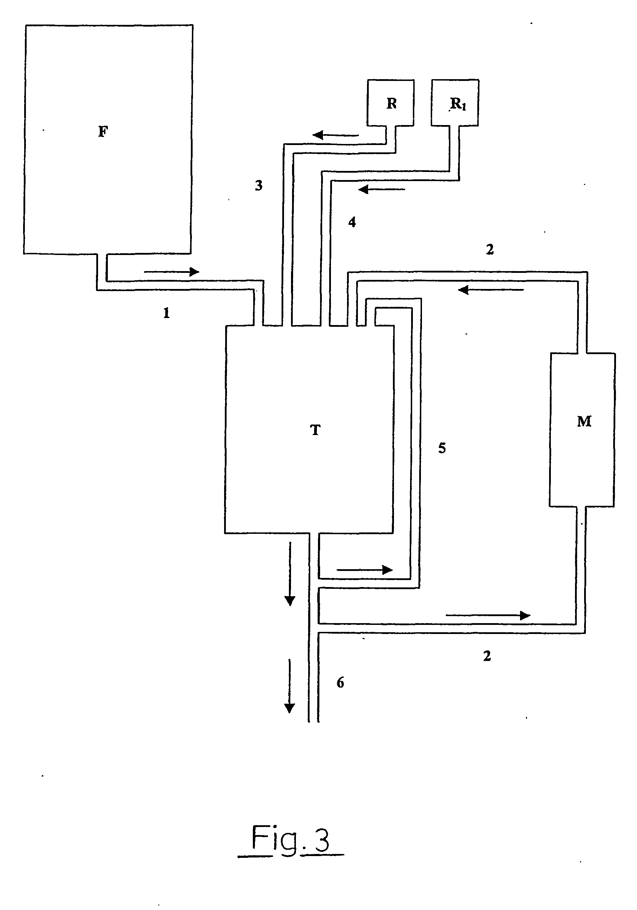

In fig. 3 such an integrated system is shown in principle. Central in the system is a multipurpose tank (T), wherein some of the processes take place, and whereto and wherefrom liquids and suspensions are transferred, one-way or in circulations. Any circulation of the contents of the multipurpose tank (T) through external loops (2, or 5) may be done continuously or step-wise and may include a number of passages (at least one) through the loop.

The following process steps and transfers between parts of the apparatus are performed.

In a fermentor (F), bacteria are cultivated normally to late logaritmic or stationary growth phase. The choice of growth medium was described above and lies well within the competence of the person skilled in the art.

When the preferred growth phase has been reached, growth medium and bacterial cells are in the integrated system transferred to the multipurpose tank (T) for further processing. Transfer from the fermentor to the tank (T) is done via conduits/tubings made of a suitable material, e.g. an inert and non-toxic polymer tubing or a metal conduit, e.g. stainless steel. Such materials may be chosen also for subsequent transfer lines and circulations or reintroduction conduits/tubings. The conduit/tubing leading from the fermentor tank to the multipurpose tank (T) has been given the reference number 1 in Fig. 3.

After transfer of bacterial suspension to the tank (T), the suspension is passed further in a circulation through a conduit/tubing (2) via a membrane filter unit (M) and reintroduced into the tank (T). In the filter unit (M) a tangential or cross flow filtration process is removing excess liquid of the medium. A more concentrated bacterial suspension is then reintroduced into the multipurpose tank (T).

When the bacterial suspension has been concentrated to a predetermined degree, a valve governing the flow in the membrane loop (2,M) is closed and a buffer is introduced into the multipurpose tank (T) to adjusting the pH in the concentrated medium to a predetermined value suitable for forming stable outer membrane vesicles. The buffer, often a high pH buffer, is introduced through a conduit/tubing (3) from a reservoir tank (R).

When the predetermined pH has been reached, the valve governing the flow from the reservoir tank (R) is closed, and mixing to uniformity of the contents of the tank (T) is obtained by circulating the suspension via a conduit/tubing (5) (the flow governed by a valve) through the tank (T) for a predetermined period of time, and the valve subsequently closed.

Thereafter, a valve governing the flow in the conduit/tubing (4) from a second reservoir tank (R₁) is opened, and a predetermined volume of the extraction buffer, containing *inter alia* a detergent, is pumped into the tank (T). The valve is then closed, and again the valve governing the flow in conduit/tubing (5) is opened to circulate the content of the tank (T) through the loop for a predetermined time period of extraction.

Finally, when bacteria have been inactivated and extracted, the contents of the multipurpose tank (T) is exited through an exit conduit/tubing (6) for further treatment, such as removal of bacterial debris and isolation and purification of OMV, outside the inetegrated system.

At any stage in the integrated process, during use of the integrated system, chemical, microbiological or physical parameters, such as for example pH, may be monitored by mounted in-line equipment, or samples may be manually or automatically withdrawn for off-line analysis.

The integrated system disclosed supra represents an aspect of the present invention to provide a stepwise production system for composition including OMVs according to the present invention.

The integrated way of processing bacteria, from bacterial growth to non-infectious crude OMV, is a new and advantageous part of an overall procedure for preparation OMV for use in vaccines according to the invention, and may be used also for preparation of other vaccines, or treatment of hazardous microbiological material for other purposes.

OMV-enrichment by centrifugation. For the detergent-treated and thus inactivated bacteria, simple centrifugation may be performed without risk, and thus the suspension may be drained from the integrated system described above, when such an integrated system has been used. At intermediate centrifugal forces, e.g. at some 10,000 to 20,000 x g, preferred about 15,000 x g for about 1 - 2 h, bacterial residue and major particulate matter can be removed, and the OMV-enriched supernatant collected for purification.

### Purification of OMV by high speed (or ultra)centrifugation.

OMV may be sedimented from the OMV-enriched supernatant by one, or preferentially more, high speed centrifugations or ultracentrifugations. Concurrently with the OMV concentration, the centrifugations will reduce the amount of detergent, and of released LPS which does not sediment in the presence of detergent. The stability of OMV may at a later stage be increased by adding a viscosity enhancer, e.g. 20% sucrose, to the centrifugation medium.

Typically, the following high speed centrifugation (or ultracentrifugation) steps may be used in order to obtain pure and stable OMV:
a. OMV-enriched supernatant is centrifuged at 4°C, e.g. at 40,000 x g for 10 h or at 100,000 x g for about 2 h;
b. the supernatant is removed and the OMV-enriched pellet is redispersed by vigorous, magnetic stirring at ambient temperature at non-foaming conditions in a dispersing solution containing 50 mM Tris buffer pH 8.7 containing 2 mM EDTA, 20% sucrose added as stabiliser, and a bile acid salt detergent, e.g. about 1% DOC, until the pellet has been fully redispersed; this may typically take 5 h;
c. the dispersion is optionally ultrasonicated for 1 to 5 min to ensure extensive microdispersion;
d. step a is repeated, with centrifugation e.g. at 40,000 x g, with time optionally shortened e.g. to 5 hours;
e. step b is repeated with the vigorous stirring until pellet dissolution, followed by weak stirring over night in aqueous 3% sucrose.

### Sterile filtration of purified OMV.

Sterile filtration cannot easily be done by filtering directly through a classical 0.22 µm filter, as clogging of the filter will rapidly occur. Such obstacles have caused several vaccine producers to give up such a step.

One or more pre-filtration steps may advantageously be tried. However, using a sequence of one 0.45 µm and one 0.22 µm filter causes considerable problems, and even such a sequence may need to be preceded by a more coarse prefilter. In our hands, a first prefilter of 1.2 µm pore size, which has been tried by others, has shown to be less efficient than a 0.8 µm prefilter. Apparently the combination of 0.8 µm + 0.45 µm will give less extensive clogging of the 0.22 µm filter. According to our experience, a series of three separate filters with decreasing pore sizes may advantageously be used, e.g. the sequence 0.8 µm + 0.45 µm + 0.22 µm filters. The filters should be used separately, not as a cassette of filters with different pore sizes, *inter alia* to allow for an individual change of filters of a given pore size, when indications of clogging can be seen, e.g. by monitoring of filtration rate or pressure.

Before the initial prefiltration of the ultracentrifuged OMV preparation, e.g. through a filter of 0.8 µm pore size, the OMV preparation is advantageously stirred e.g. over night at room temperature, and ultrasonicated e.g. for 1 min to dissociate aggregates that may have been formed in or after the previous step.

Filters should be chosen with care. Especially the larger vesicles will during filtration experience shear forces, which may vary with pore size and filter quality, e.g. such as surface characteristics. A too rugged surface may be harmful for the OM vesicles. Some filters have a rather rugged pore surface structure as seen by EM. It is our experience that 0.2 um polyethersulphone filters yield vesicles with less rupture and damage, and in higher yield, than corresponding polyvinylidene fluoride filters.

As OMV form spontaneously in sizes predominantly with diameters in the range of 50 to 200 nm, a conventional 0.22 µm sterile filter may be just marginally applicable for filtration of OMV. Vesicles in the upper range may, however, be just capable to pass filters of this size, due to OMV flexibility which allows a reshaping during filtration with accompanying passage through somewhat smaller pore size than theoretically expected.

Filtration outcome will also be influenced by the temperature applied during filtration. For the larger vesicles, having diameters close to the filter pore size, the OMV flexibility may be important for their capability to pass the filter. As for other phospholipid- and protein-containing membranes, the flexibility of the vesicular membranes can be strongly influenced by temperature, e.g. due to the presence of acyl chains of phospholipids or by the lipophilic part of deoxycholate.

Influence by temperature on the filtration process has been shown by filtration of OMV through a 0.45 um filter at 4 °C and 40 °C. Clogging was substantially increased at the lower temperature, and thus we recommend that filtrations be done at ambient temperature or above, in order to avoid the occurrence of excessively stiff vesicles, with corresponding risk of OMV damage or loss.

Generally it may be noted that centrifugation or ultracentrifugation is best done at lower temperature, e.g. at 4-6 ° C. After collecting the pelleted material, this must be carefully redispersed to remove aggregates formed, and components trapped in the aggregates. This redispersion should therefore be done at higher temperature, e.g. at room temperature, and for more extended periods of times, e.g. such as some 5 hours or over night, in order to be efficient.

Mechanical stirring of OMV can be done with less caution when performed in presence of a stabilising compound. For this reason, for example after the first centrifugation, concentrated sucrose may be added, e.g. to 20%. After the last centrifugation, when sucrose concentration is less, e.g. 3%, only weak stirring at a more prolonged period of time should be performed.

Ultrasonication for 1 to 5 minutes, typically 1 min, is routinely used to redisperse OMV pellets after the the high speed centrifugations, and thus before sterile filtration. This is a done as a precaution, and it has been shown that even more extensive ultrasonication before sterile filtration will not change the yield or purity of the final product.

The purified and stabilised OMV may be used as a vaccine as such, but may be more immunogenic when used in combination with an adjuvant, e.g. being adsorbed to Al(OH)₃ using techniques known in the art.

The process for producing an OMV vaccine including non-denatured proteins has been disclosed supra under reference to *N*. *meningitidis.* However, a process corresponding to the one disclosed is applicable also with other Gram-negative bacteria having outer membrane that can be disaggregated by the mild detergent method, since the method is aimed at obtaining outer membrane vesicles having proteins in their original non-denatured conformation, providing antigenic determinants to be used in vaccines against the relevant bacterial strain.

Additionally, the OMVs produced by the process according to the present invention, may when included in a vaccine be provided with commonly known adjuvants, carriers, enhancers and anti-bacterial agents as well as compounds stabilising their conformation or enhancing their shelf-life. Such compounds or compositions are known for the person skilled in the art.

According to the present invention there has, by investigating a representative collection of serogroup A meningococcal strains, been shown that it may be possible to produce an outer membrane vesicle vaccine against infections with *N. meningitidis* serogroup A, aiming at giving a broad protection against the relevant strains within this serogroup. Thus, analysis of the antibodies produced in sera of mice immunised with the relevant OMV vaccines showed by immunoblotting that they were mainly directed towards the major outer membrane proteins. Notable is that we found strong antibodies towards PorA, which is generally regarded to be an important antigen of serogroup A strains. Important is also that the sera were found to be bactericidal when tested with human complement against all serogroup A strains. Although e.g. rabbit complement has been reported to give higher titers, the positive response using human complement is of significant importance.

The meningococcal serogroup A OMV vaccine according to the invention is thus a promising candidate for a human vaccine that may give an overall protection against serogroup A strains, not least including the epidemic/pandemic meningococcal strains occurring in the "Meningitis Belt" of Africa.

Generally, the process according to the present invention concerns a process for producing an outer membrane vesicle vaccine from Gram-negative bacteria comprising the steps of
a) cultivating the bacterial cells;
b) concentrating the cultivated cells from step a);
c) treating the cells with a bile acid salt detergent at a pH sufficiently high not to precipitate the detergent, for inactivating the bacteria, disrupting the outer membrane of the bacteria and forming vesicles of the outer membrane of the bacteria, said vesicles comprising outer membrane components mainly presented in their native form;
d) centrifuging the composition from step c) at 10,000 - 20,000 x g for about 1 to 2 hours to separate the outer membrane vesicles from the treated cells and cell debris, and collecting the supernatant;
e) performing a high speed centrifugation of the supernatant from step d) and collecting the outer membrane vesicles in a pellet;
f) redispersing the pellet from step e) in a buffer by stirring at ambient temperature;
g) performing a second high speed centrifugation in accordance with step e), collecting the outer membrane vesicles in a pellet;
h) redispersing the pellet from step g) in an aqueous medium containing a stabilising agent by stirring at ambient temperature;
i) performing a step-wise sterile filtration through at least two filters of decreasing pore size of the redispersed composition from step h), ending with a filter of pore-size of about 0.2 µm,
j) optionally including the composition from step i) in a pharmaceutically acceptable carrier and/or adjuvant composition.

Alternatively, the process indicated supra may be performed wherein an extra ultrasonication step is performed subsequently to step h), but prior to step i) and/or optionally subsequently to step f), but prior to step g).

Referring to Fig. 3, the invention includes also an apparatus suitable for producing outer membrane vesicles from a medium containing Gram negative bacteria, wherein the apparatus includes a fermentor (F) for cultivating the bacteria to a bacterial suspension, the fermentor (F) being connected via a conduit/tubing (1) to a multipurpose tank (T), the tank (T) being connected via conduits/tubings (3,4) to a number of auxiliary reservoirs (R,R₁) containing liquids for treating the bacterial suspension introduced into the multipurpose tank (T), and wherein the multipurpose tank (T) further is equipped with conduits/tubings (2) leading to a membrane filter unit (M) for concentrating the bacterial suspension in the multipurpose tank (T) by tangential or cross flow filtration, said tank (T) further being equipped with a exit conduit/tubing (6) for draining off of the treated contents of the multipurpose tank (T).

In the apparatus indicated supra, the multipurpose tank (T) may further be provided with a reintroduction loop (5) for circulating the contents in the multipurpose tank.

The following examples serve to illustrate the invention.

### Example 1. Vaccines from outer membrane vesicles (OMV) of meningococcal serogroup A strains grown in shake culture

Two *N*. *meningitidis* serogroup A strains of African origin (MK83/94, from Mali; MK100/97, from Togo) were chosen as representative strains of the African epidemic situation. Briefly, bacteria were grown on agar plates, transferred to a shake culture, and outer membranes (OM) were extracted with DOC. OMV was then isolated and purified by several steps of (high speed) centrifugation.

### a) Cultivation of bacteria in shake cultures

The two meningococcal serogroup A strains were initially grown on Tryptic Soy Agar (TSA) plates with VITOX (a mixture of essential growth factors for N. meningitidis) over night in a 5% CO₂/air cabinet at 35°C. The strains were again grown, each strain on 4 TSA plates with VITOX, and bacteria were after about 8 hours collected into tubes with 5 ml of pre-warmed Frantz' medium, and finally smooth suspensions of bacteria were made. The suspensions were added as inocula to Fernbach bottles with 1.4 1 of Frantz' medium.

Cultivation of the bacteria was performed with shaking (135 rpm) at 35 °C for about 10 hours. OD_{λ650nm} was determined for a 1:5 dilution in growth medium, and bacteria were collected by centrifuging at 6,000 rpm for 15 minutes.

### b) Preparation of OMV by deoxycholate (DOC) extraction.

To the bacterial cell pellets harvested from shake cultures (Example 1a) 5 ml of a first buffer (0.1 M Tris-HCl with 10 mM EDTA and 0.1% sodium thiomersal, pH 8.6) was added per gram wet weight of bacteria. After resuspension in a homogeniser, the material was retransferred to the centrifuge bottle, followed by addition of 0.05 ml of a second buffer (10% DOC in the first buffer, pH 8.9) per ml of the first buffer, to give a final DOC concentration of 0.5%. Then suspensions were DOC-extracted by magnet stirring (200 rpm; 30 min) at ambient temperature. The extracted suspensions were centrifuged (6,000 rpm; 15 minutes), the supernatants were centrifuged cold (15,000 rpm; 4°C; 30 min), and supernatants were centrifuged again (15,000 rpm; 4°C; 15 min) and the OMV-containing supernatants stored cold.

The supernatants were high speed centrifuged (40,000 rpm, corresponding to 125,000 x g; 4 °C; 90 min; "45Ti" rotor). The pellets were resuspended in 6 ml of buffer (0.05 M Tris-HCl pH 8.9; 2 mM EDTA, 2.5% DOC). To these suspensions were added 12 ml of another buffer (0.05 M Tris-HCl pH 8.9; 2 mM EDTA; 0.5 % DOC; 30% sucrose and 0.01% sodium thiomersal), followed by homogenisation. At this stage sucrose was in about 20% concentration, and DOC at about 1.2%. After an ultrasonic treatment for 1 minute, the suspensions were again ultracentrifuged as above (140,000 x g; rotor "50.2Ti"). The pellets were resuspended in a total of 10 ml of 3 % sucrose with 0.01% sodium thiomersal. The OMV suspension was then ultrasonicated for 1 minute and stored at 4 °C.

### c) Characterization of the OMV preparations

OMV were by immunoblot technique shown to contain the relevant proteinaceous antigenic determinants, PorB, PorA, Rmp and Opc. The specificities were as expected from the whole cell analysis (see Table 1).

LPS pattern of the OMV preparations were established using a SDS-PAGE technique with silver staining for carbohydrate. Quantification of LPS by a semiquantitative technique revealed that LPS contents in OMV were strongly reduced relative to protein (5-10%), compared to whole cell preparations. Further, the LPS immunotype was found to be L3,7,9, using an immunoblot technique with reference monoclonal antibodies.

Electron microscopy (EM) revealed OMV vesicles in the size of 70 to 170 nm.

Thus the production method provided a vaccine product apparently with a native antigen mosaic and a strongly reduced level of LPS.

### d) Adsorption of OMV to the adjuvant Al(OH)₃

Before adsorption to the Al(OH)₃ adjuvant, the protein content of the OMV preparation was determined, and the preparation diluted with 3% sucrose to a protein concentration of 0.1 mg/ml.

To obtain the final vaccine preparations (50 µg protein/ml vaccine), "Superphos Alhydrogel" containing 30 mg/ml Al(OH)₃ was diluted with 3% sucrose (with 0.01% sodium thiomersal) to an Al(OH)₃ concentration of 6.6 mg/ml, and mixed with an equal volume of OMV. Adsorbtion was effected by magnetic stirring over night at ambient temperature. The Al(OH)₃-adsorbed OMV preparation was stored at 4 °C until tested as vaccines to prove their efficiencies.

### Example 2. Preparation of an OMV vaccine from a meningococcal serogroup B strain 44/76 grown in a fermentor

### a) Production and purification of OMV

Cultivation of *N. meningitidis* 44/76 was initiated on growth on 6 Tryptone Soy Agar plates at 35 °C in 5 % CO₂/air atmosphere for 12 h. Cells were harvested into 3 tubes with 5 ml Frantz' medium. Contents of the tubes were added to 3x 1 l flasks containing Frantz' medium and grown by shaking over night to yield the inoculate. This was added to a Bioengineering LP351 fermentor with 75 1 capacity, containing 51 1 of pre-sterilised Frantz' modified medium and sterile-filtered yeast extract dialysate. The pH after inoculation was 7.06, falling to 6.39 during cultivation for 9 h at 36 °C with stirring and aeration. Oxygen once had to be substituted for air when level of dissolved O₂ approached zero. Growth was terminated at an OD_{λ650nm} of 6.36, the fermentor was cooled to 6 °C by a cooling system, the air supply was closed, and stirring continued at 100 rpm over night.

Transfer of the bacterial suspension from the fermentor was done by stepwise pumping to a Millipore CUF 5/4/1 cross flow filtration (CFF) unit equipped with valves, pumps and a filter module with 2 Pellicon P2B300V05 polyethersulphone filters (300 kD cutoff). Initial transfer of 20 l bacterial suspension was followed by small portions until the fermentor was emptied.

Concentrating the suspension was performed in the CFF unit by circulating the suspension to be passing by the filters, with the transmembrane pressure being continuously monitored and kept < 0.5 bar (observed: 0.24 - 0.33 bar). As the transmembrane pressure at 45 min rapidly rose above 1 bar, the concentrating was terminated. A weighing cell disclosed a residual volume of close to 3 1 of concentrate.

Adjustment of pH of the concentrated bacterial suspension from pH 6.24 to 8.1 was done by adding, via a tubing system, 5 1 of a 0.1 M Tris-HCl buffer of pH 9.0 with 10 mM EDTA, followed by 15 min stirring in the CFF unit to secure uniform conditions.

Inactivation/Extraction of OM material was initiated by adding, via tubing, 400 ml of an 0.1 M Tris-HCl buffer (pH 8.9) containing 10 % DOC, to give a final DOC concentration of 0.5%. Subsequently the suspension was circulated in the CFF unit for 30 min, without contact with the filters. The extracted suspension (9 1), checked to be completely without living bacteria, was drained off by pumping into a flask and kept at 4 °C.

Preparation of crude OMV was done by distributing the inactivated suspension to centrifuge tubes of 500 ml, and centrifuging in a Sorvall RC-26 PLUS centrifuge at 15,000 g for 90 min at 4 °C in, collecting 8.0 1 of supernatant.

Purification of crude OMV was done by high speed centrifugation twice. The supernatant was distributed to centrifuge tubes of 250 ml, and centrifuged in a Centrikon T-1170 high speed centrifuge at 40,000 g for 10 h at 4 °C. The pellets were resuspended in altogether 1.1 1 of 50 mM Tris-HCl buffer pH 8.8 with 2 mM EDTA and 2.5% DOC, and then was added 2.2 1 of another buffer (50 mM Tris-HCl pH 8.6 with 2 mM EDTA and 0.5% DOC) containing 30% sucrose. Final concentrations were then approximately 1.2% DOC and 20% sucrose. The suspensions were magnet stirred at room temperature at high speed, below the point of foaming. When completely homogeneous after 10-12 h, the portions of the suspension were treated by ultrasound for 1 min (50 W; 35 kHz; Elma Transsonic 420 apparatus).

A second high speed centrifugation was performed for 5 h at 4 °C, the pellets were resuspended in altogether 825 ml of 3% sucrose with magnetic stirring at room temperature until homogeneous, and treated by ultrasound as above.

Final purification of OMV was performed at 20 °C by filtering through three capsule filters (Gelman Science Suporlife DCF) in sequence, first two prefilters of 0.8 um and 0.45 um, respectively, then adjustment of the protein concentration to 1.2 mg/ml, before the final sterile filtration (0.22 um), with an overall yield of 1.22 1; the OMV protein concentration was 0.68 mg/ml.

### b) Adsorption of OMV to the adjuvant Al(OH)₃

The purified OMV preparation was used for the preparation of the final a vaccine by adsorption of OMV to Al(OH)₃. This was done by diluting 1.2 1 of the purified OMV preparation to 0.2 mg protein /ml with 2.9 1 of 3% sucrose, adding with stirring of 4.2 kg of a dispersion of Al(OH)₃ (13 mg/ml) in 3% sucrose, and finally diluting with 8.3 1 of 3% sucrose, yielding 16.6 kg of adsorbed vaccine.

### c) Characterization of the serogroup B vaccine

*Contents of one human vaccine dose (0.5 ml)*

| | | |
|---|---|---|
| OMV protein | | 25 ug |
| LPS | | 1.8 ug |
| Deoxycholate | | 6.5 ug |
| DNA | | 0.7 ug |
| Aluminium | | 0.6 mg |
| Sucrose 3% | ad | 0.5 ml |

### Antigens demonstrated in the OMV preparation

OM proteins (by SDS-PAGE and immunoblot):

| | |
|---|---|
| 80 kD | (Omp 85) |
| 70 kD | (FrpB) |
| class 1 | (P1.7; P1.16) |
| class 3 | |
| class 4 | |
| class 5 | (Opa; Opc). |

LPS immunotypes (by SDS-PAGE and silver staining):
L3, 7, 9; and L8

### Example 3. Preparation of an OMV vaccine from a meningococcal serogroup A strain

Meningococcal strain MK83/94 (Mali) is grown in a fermentor in Frantz' medium until early stationary growth, and OMV isolated and purified by the process of Example 2. OMV is characterized and found similar to OMV of Example 1.

### Example 4. Test of two serogroup A OMV vaccines in mice serum bactericidal assay (SBA)

### a) General procedure

The efficiency of the vaccines from Examples 1 and 2, to provide protection against *N*. *meningitidis* strains was tested with the method of serum bactericidy, an assay believed to correlate with protection against meningococcal disease. A commercial polysaccharide group A+C vaccine was included as a control; such polysaccharide vaccines are known not to function well in SBA.

Sera from immunised (and non-immunised) mice were tested for their ability to kill selected bacteria. Mice used in the procedure were outbred NMRI female mice (weight 12-14 g), receiving 2 doses of vaccine with an interval of 3 weeks. Sera were obtained 2 weeks after the second immunisation.

The sera were tested for bactericidal properties against four different meningococcal test strains. In addition to the two serogroup A strains MK83/94 and MK100/97 used for preparation of vaccines (Example 1), one further serogroup A strain (MK42/98 from Burkina Faso) also representative of the "Meningitis Belt", and the Norwegian serogroup B vaccine strain 44/76 (used in example 2) were tested. The strain characteristics are shown in Table 2.

**Table 2. Characterising traits of bacterial strains used in bactericidal testing.**

| Serum Bactericidal Assay (SBA) strain | Country of origin | Antigenic determinants | Clone complex |
|---|---|---|---|
| MK83/94 | Mali | A:4,21;P1.20,9; L3,7,9 | Subgroup III-1 |
| MK100/97 | Togo | A:4,21;P1.20,9; L3,7,9 | Subgroup III-1 |
| MK42/98 | Burkina Faso | A:4,21;P1.20,9; L3,7,9 | Subgroup III-1 |
| 44/76 | Norway | B:15;P1.7,16; L3,7 | ET-5 |

### b) Vaccine preparations for production of antisera in mice, and SBA results

Sera to be used in the bactericidal assay were obtained from mice, grouped as having been injected with one of a number of vaccine preparations or combinations thereof:

### Group 01. 1 µg 44/76 OMV vaccine (serogroup B-vaccine; prepared essentially as in example 2)

Against the serogroup B strain 44/76, all of the mice provided bactericidal sera, with arithmetic mean of the log₂ titers of 10.0. Against the serogroup A strain MK83/94, 8 of the 10 mice did not respond with positive sera. 9 of 10 sera did not react against the serogroup A strains MK42/98 and MK100/97.

### Group 02. 1+1 µg serogroup A+C polysaccharide (a commercial vaccine).

Against the serogroup A strains MK83/94 and MK100/97 all mice sera were non-reacting in SBA. For the serogroup A strain MK42/98, 8 of the mice were non-responders; the two responding mice (02-4 and 02-5) both had a log₂-titer of 5.

### Group 03. 1 µg MK83/94 OMV-protein (serogroup A vaccine prepared as in example 1)

Against the serogroup A strain MK83/94 all of the mice in the group responded, with arithmetic mean for the log₂₋titers of 8.9. Of the same mice, 9 of 10 responded against both of the serogroup A strains MK42/98 and MK100/97. The arithmetic means of the log₂-titers were 7.9 and 8.1, respectively. One mouse was a common non-responder against these two group A strains. Except for one mouse (log₂₋titer 9), all of the mice in this group were non-responders against the serogroup B strain 44/76.

### Group 04. 4 µg MK83/94 OMV-protein (serogroup A vaccine prepared as in example 1)

Against the serogroup A strain MK83/94 all of the mice in this group responded, and the arithmetic mean for the log₂₋titers was 9.1. Against the serogroup A-strains MK42/98 and MK100/97 all of the mice responded, with arithmetic mean of the log₂-titers of 9.0 and 8.9, respectively. Of all the mice in the group, one mouse responded generally with the lowest log₂-titer against all of the three serogroup A-strains (the log₂-titers were alternately 7.6 and 6). Against the serogroup B strain 44/76, 3 of 10 mice responded with a log₂-titer between 6 and 10. One mouse did not respond and for the 6 last mice there was observed a partial killing of the bacteria.

### Group 05. Control group (unvaccinated)

3 of 5 mice were non-responders against the serogroup A strain MK83/94. 4 of 5 mice were non-responders against the serogroup A strains MK42/98 and MK100/97. One mouse responded against strain MK83/94 with a log₂-titer of 3, and one mouse responded against all of the three serogroup A strains in a log₂-titer between 4 and 8. Against the serogroup B strain 44/76 there was observed a partial activity for 4 of the 5 mice in the group for some dilutions.

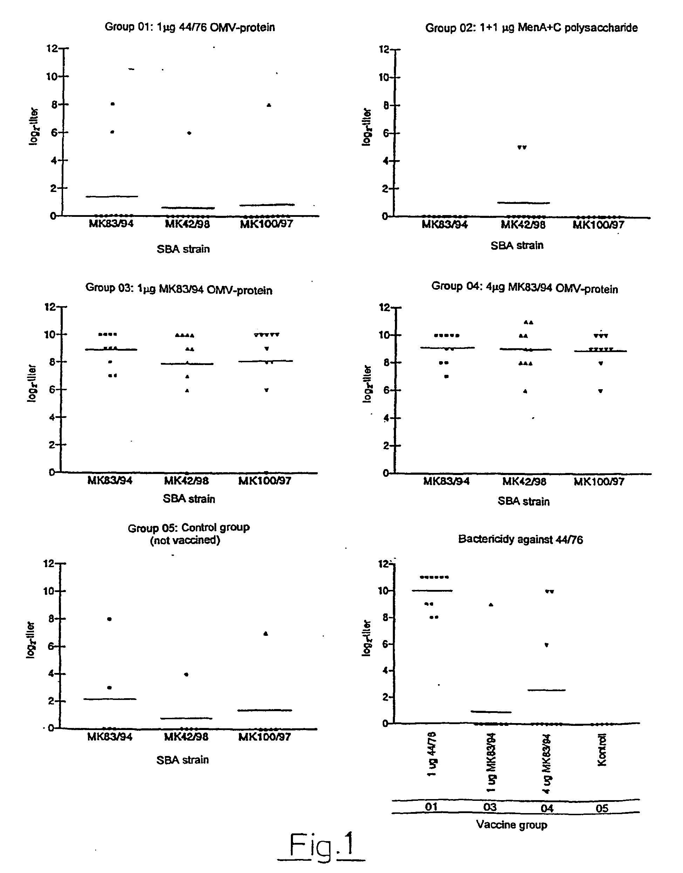

Fig. 1 shows a graphic presentation of the bactericidal properties of sera against the selected meningococcal strains, obtained from vaccinated mice.

Mice immunised with 1 µg MK83/94-OMV-protein (group 03) or with 4 µg MK83/94-OMV-protein (group 04) responded with a significantly higher log₂-titer against all of the serogroup A strains than did mice immunised with 1 µg 44/76-OMV-protein (group 01) or 1+1 µg A+C polysaccharide vaccine(p<0.05).

There was no statistically significant difference between the bactericidal efficacy against MK83/94 in sera from mice immunised with 1 µg MK83/94-OMV-protein (group 03) and sera from mice immunised with 4 µg MK83/94-OMV-protein (group 04) (p>0.05). There was not observed any corresponding statistically significant difference in the bactericidal properties between the two groups 03 and 04, neither against any of the heterologous serogroup A strains M42/98 or MK100/97, nor against the serogroup B strain 44/76 (p>0.05).

There was no statistically significant difference in bactericidal properties against the three serogroup A strains for sera from mice immunised with 1+1 µg A+C envelope polysaccharide (group 02) and sera from mice in the control group (group 05) (p>0,05). This was not unexpected, as the model is not a good one for test of bactericidal activity when mice have been immunized with pure polysaccharides.

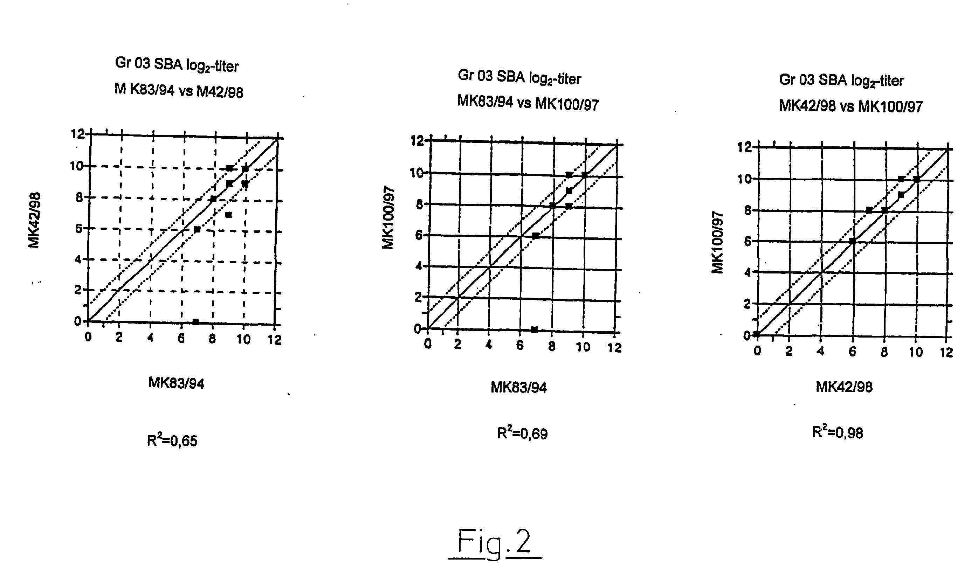

Bactericidal properties of sera from mice immunised with 1 µg MK83/94-OMV-protein (group 03) showed a good correlation for the different serogroup A strains against each other. Thus, a cross plot of the bactericidal properties against the different serogroup A strains (Fig. 2) shows that those sera which have high log₂-titers towards strain MK83/94 also have high log₂-titers towards the two other serogroup A strains.

One conclusion of these tests is that the OMV-vaccine according to the present invention may give an overall protection against different types of serogroup A meningococci, as tested in mice.

### Example 5. Filtration of OMV at 6 °C and 40 °C

A preparation of OMV was prepared as in Example 2, including purification by two high speed centrifugations and stabilisation in 3% sucrose. This preparation was prefiltered with two filters in sequence, pore sizes of 1.2 um and 0.8 um, respectively, and subsequently divided in two parts of about 35 ml.

One part was filtered through a 0.45 um (Millex HA) filter, taken directly from the storage temperature of 4 °C; the actual temperature during filtration was about 6 °C. The other part was first carefully warmed to about 40 °C before filtering.

Yields of protein were about the same for filtrates obtained by the two temperatures. However, during the 4-6 °C filtration the final 0.45 um filter had to be changed 4 times due to clogging, in contrast to only one filter change being required when filtering was performed at 40 °C.

### Example 6. Filtration of OMV at various pore size combinations

OMV (Example 2) was filtered through two different sequences of polyethersulphone (PES) filters:
0.8 um -----→ 0.45 um ---→ 0.22 um: overall yield 67%
0.8 um --------------------→ 0.22 um: overall yield 38%

Thus, omission of the 0.45 um filter substantially reduced the overall yield after the final 0.22 um sterile filtering.

### Example 7.Inactivation of concentrated meningococcal cultures in Tris buffer containing DOC

### a) Inactivation in 0.16% DOC of cultures previously concentrated by centrifugation

A 50 1 batch of *N*. *meningitidis* 44/76 (serogroup B) was grown in a fermentor in Frantz' modified medium at 36 °C until early stationary phase (as for Example 2). Final OD values of 6.42 and viable counts (CFU/ml) of 7.4 x 10⁹, were measured. Aseptically, two 65 ml samples were withdrawn, stored overnight at room temperature, and concentrated by centrifugation at 30,000 rpm for 20 min at 4 °C in a Beckman Coulter^{™} "Avanti J-251" centrifuge. To each of the pellets were added about 19 ml (5 ml per g wet weight) of a buffer, thus corresponding to a concentrating factor of about 3.4 times for the fermentor-grown bacterial suspension. The buffers were for both samples a 0.1 M Tris-HCl buffer with 10 mM EDTA, of pH values 8.0 and 8.6, respectively. After addition of the buffers, bacterial cells were resuspended by gentle shaking for 8 min. No inactivation could be found at this stage.

Then 20 ul of the same buffer, with 10% DOC, was added per ml of the first buffer with brief stirring to a final concentration of 0.16 % DOC and the samples were left for 15 min for the detergent to acting.

Aliquots were taken for revealing of any surviving meningococci in two tests: 1) dilution of 5 ml of bacterial suspension into 250 ml Tryptone Soy Broth, which will be diluting away and abolishing the effect of DOC; 2) using 100 ul to inoculate blood agar plates. For both tests, media were checked for growth after 7 days at 30-36 °C in a 5% CO₂/air atmosphere. None of the samples showed any living meningococci, and thus inactivation was complete with slightly less than 0.2% DOC for 15 min at either pH 8.0 or 8.6.

The experiment was repeated with another 50 1 batch where cultivation was stopped at an OD of 6.36, and viable counts of 2.0 x 10¹⁰ . Similar results were obtained.

### b) Inactivation in buffers containing 0.5% DOC of cultures previously concentrated by cross flow filtration (CFF)

In our laboratory, about 50 batches of meningococcal strains of serogroup B have been prepared using 0.5% DOC for inactivation of bacteria/extraction of OM on cultures concentrated by crossflow filtration (Example 2), without visible growth in any case.

### c) Inactivation in Tris buffers with various concentrations of DOC - of cultures previously concentrated by CFF

Meningococcal strain 44/76 (serogroup B) is grown in a fermentor in Frantz' medium, concentrated about 10 times in a CFF unit, added about 5 1 of 0.1 M Tris pH 9.0 with 10 mM EDTA, and circulated for 15 min (Example 2). Aseptically, 1 l concentrated pH-adjusted culture is pumped into a flask.

Equal portions of 45 ml of the concentrated and pH-adjusted culture are added 5 ml of 0.1 M Tris-HCl pH 8.9 with 10 mM EDTA, also containing DOC in amounts giving a series (with duplicates) of final DOC concentrations ranging from 0.5% to 0.025%.

Samples with complete inactivation of viable cells are further purified with the OMV procedure of Example 2. OMV quality is checked by electron microscopy, and OM proteins characterised to establish a low concentration of DOC that simultaneously will kill the cells and provide OMV of a high quality.

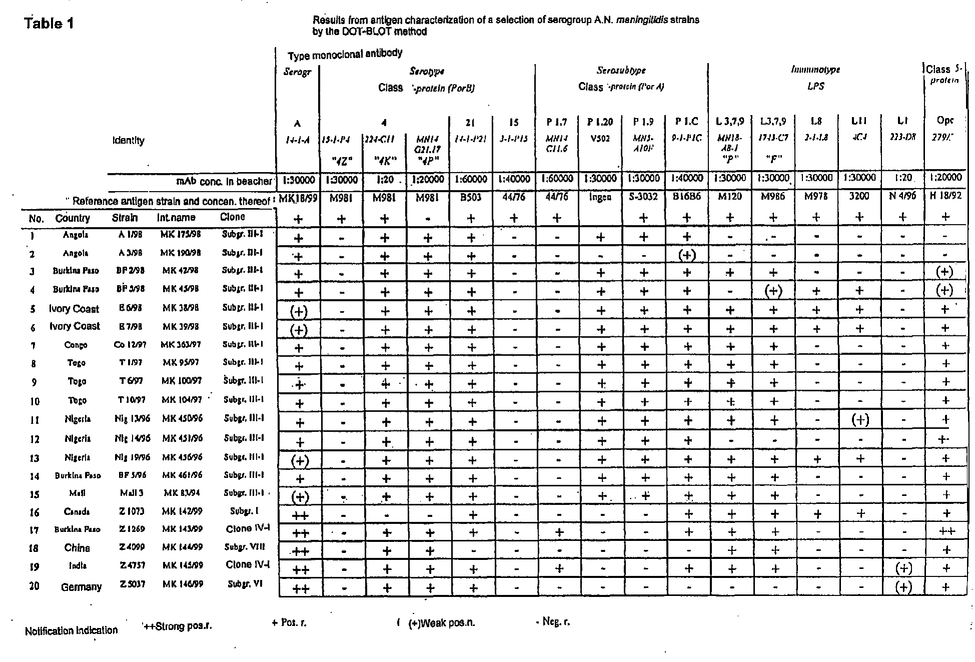

## Claims

1. A vaccine directed against meningococcal bacteria of serogroup A, **characterised in that** it comprises proteinaceous outer membrane vesicles from the bacteria wherein the protein components are present mainly in a native conformation, optionally together with pharmaceutically acceptable carriers and/or adjuvants.

2. A vaccine according to claim 1, **characterised in that** the outer membrane proteins comprise the proteins Por B (P3.4 and P3.21) and Por A.

3. A vaccine according to claim 2, **characterised in that** the Por A proteins are P1.9 and P1.20.

4. A vaccine according to claim 2 or claim 3, **characterised in that** the outer membrane proteins further comprise Opc.

5. A process for producing the vaccine of claim 1, comprising cultivating the meningococcal bacteria in a fermentor, followed by extraction of the membrane vesicles with deoxycholate so as to form outer membrane vesicles.

6. A process for producing the vaccine of claim 1, comprising cultivating the meningococcal bacteria in a fermentor, followed by extraction of the membrane vesicles with a bile acid salt detergent so as to form outer membrane vesicles, wherein the process is performed in a closed, integrated system comprising a fermentor and a multipurpose treatment tank connected to a membrane filtration module.

7. The process of claim 6 which comprises the steps of
a) cultivating the cells of said bacteria in said fermentor;
b) transferring the cell suspension to said multipurpose tank;
c) concentrating the cell suspension in a loop from the multipurpose tank by cross flow tangential filtration;
d) adding under stirring a buffer to the multipurpose tank for adjusting pH to a value sufficiently high not to precipitate said bile acid salt detergent;
e) treating the cells with said bile acid salt detergent so as to inactivate the bacteria, disrupt the outer membrane and form vesicles of the outer membrane of the bacteria, said vesicles comprising outer membrane components mainly presented in their native form; and
f) draining off the non-infectious outer membrane vesicle-containing bacterial extract.

8. The process of claim 7 which further comprises
a) enriching the resulting bacterial extract by separating the outer membrane vesicles from treated cells and debris;
b) performing a high speed centrifugation of the supernatant from step (a) and collecting the outer membrane vesicles in a pellet;
c) redispersing the pellet from step (b) in a buffer by stirring at ambient temperature;
d) performing a second high speed centrifugation in accordance with step (b) and collecting the outer membrane vesicles in a pellet;
e) redispersing the pellet from step (d) in an aqueous medium containing a stabilising agent by stirring at ambient temperature;
f) performing a step-wise filtration through at least two filters of decreasing pore size of the redispersed composition from step (e), ending with a filter pore size of about 0.2 mm; and
g) optionally including the composition from step (f) in a pharmaceutically acceptable carrier and/or adjuvant composition.

9. Apparatus suitable for producing outer membrane vesicles from a medium containing Gram negative bacteria, wherein the apparatus includes a fermentor (F) for cultivating the bacteria to form a bacterial suspension, the fermentor (F) being connected by a conduit/tubing (1) to a multipurpose tank (T), the tank (T) being connected via conduits/tubings (3,4) to a number of auxiliary reservoirs (R, RI) containing liquids for treating the bacterial suspension introduced into the tank (T), and wherein the tank (T) further is equipped with conduits/tubings (2) leading to a membrane filter unit (M) for concentrating the bacterial suspension in the tank (T) by tangential or cross flow filtration, said tank (T) further being equipped with a reintroduction loop (5) for circulating the contents in the tank, and the tank (T) further being equipped with an exit conduit (6) for draining off the treated content from the tank (T).

10. Use of the apparatus of claim 9 in the preparation of a proteinaceous outer membrane vesicle vaccine against a disease caused by Gram negative bacteria.

11. The use according to claim 10 wherein said Gram negative bacteria is *Neisseria meningitidis* serogroup A.

## Patentansprüche

1. Vakzin, das gegen Meningokokkusbakterien der Serogruppe A gerichtet ist, **dadurch gekennzeichnet, dass** es Vesikel der proteinhaltigen äußeren Membran der Bakterien umfasst, wobei die Proteinkomponenten überwiegend in nativer Konformation vorliegen, gegebenenfalls zusammen mit pharmazeutisch verträglichen Trägern und/oder Adjuvantien.

2. Vakzin nach Anspruch 1, **dadurch gekennzeichnet, dass** die Proteine der äußeren Membran die Proteine Por B (P3.4 und P3.21) und Por A umfassen.

3. Vakzin nach Anspruch 2, **dadurch gekennzeichnet, dass** die Por A-Proteine P1.9 und P1.20 sind.

4. Vakzin nach Anspruch 2 oder Anspruch 3, **dadurch gekennzeichnet, dass** die Proteine der äußeren Membran außerdem Opc umfassen.

5. Verfahren zur Herstellung des Vakzins nach Anspruch 1, umfassend die Kultivierung der Meningokokkusbakterien in einem Fermentor, gefolgt von der Extraktion der Membranvesikel mit Desoxycholat unter Bildung von Vesikeln der äußeren Membran.

6. Verfahren zur Herstellung des Vakzins nach Anspruch 1, umfassend die Kultivierung der Meningokokkusbakterien in einem Fermentor, gefolgt von der Extraktion der Membranvesikel mit einem Gallensäuresalz-Detergenz, unter Bildung von Vesikeln der äußeren Membran, wobei das Verfahren in einem geschlossenen, integrierten System durchgeführt wird, das einen Fermentor und einen Vielzweck-Behandlungstank, verbunden mit einem Membranfiltrationsmodul umfasst.

7. Verfahren nach Anspruch 6, umfassend die folgenden Schritte:
a) Kultivierung der Bakterienzellen in dem Fermentor;
b) Überführung der Zellsuspension in den Vielzwecktank;
c) Aufkonzentrieren der Zellsuspension in einer Schleife des Vielzwecktanks durch Tangentialflussfiltration;
d) Zugabe eines Puffers unter Rühren in den Vielzwecktank, um den pH auf einen Wert einzustellen, der ausreichend hoch ist um das Gallensäuresalz-Detergenz nicht auszufällen;
e) Behandlung der Zellen mit dem Gallensäuresalz-Detergenz, um die Bakterien zu inaktivieren, die äußere Membran zu zerstören und Vesikel der äußeren Membran der Bakterien zu bilden, wobei die Vesikel Komponenten der äußeren Membran überwiegend in ihrer nativen Form umfassen; und
f) Ableiten des nicht-infektiösen, Vesikel der äußeren Membran enthaltenden bakteriellen Extraktes.

8. Verfahren nach Anspruch 7, welches weiterhin umfasst:
a) Anreicherung des resultierenden bakteriellen Extraktes durch Abtrennung der Vesikel der äußeren Membran von behandelten Zellen und Zellfragmenten;
b) Durchführung einer Hochgeschwindigkeitszentrifugation des Überstandes aus Schritt (a) und Sammeln der Vesikel der äußeren Membran in einem Pellet;
c) Redispergieren des Pellets aus Schritt (b) in einem Puffer durch Rühren bei Umgebungstemperatur;
d) Durchführung einer zweiten Hochgeschwindigkeitszentrifugation in Übereinstimmung mit Schritt (b) und Sammeln der Vesikel der äußeren Membran in einem Pellet;
e) Redispergieren des Pellets aus Schritt (d) in einem wässrigen Medium, enthaltend eine Stabilisierungsagens durch Rühren bei Umgebungstemperatur;
f) Durchführung einer stufenweisen Filtration der redispergierten Zusammensetzung aus Schritt (e) durch wenigstens zwei Filter mit abnehmender Porengröße, endend bei einer Filterporengröße von etwa 0,2 mm; und
g) gegebenenfalls die Aufnahme der Zusammensetzung aus Schritt (f) in einem pharmazeutisch verträglichen Träger und/oder einer Adjuvanszusammensetzung.

9. Apparatur, geeignet zur Herstellung von Vesikeln der äußeren Membran aus einem Medium enthaltend gramnegative Bakterien, wobei die Apparatur umfasst: einen Fermentor (F) zur Kultivierung der Bakterien zur Bildung einer Bakteriensuspension, wobei der Fermentor (F) über eine Leitung/Rohr (1) mit einem Mehrzwecktank (T) verbunden ist, wobei der Tank (T) über Leitungen/Rohre (3, 4) mit einer Anzahl von Hilfsreservoirs (R, RI) verbunden ist, welche Flüssigkeiten zur Behandlung der in dem Tank (T) eingeführten Bakteriensuspension enthalten, und wobei der Tank (T) außerdem mit Leitungen/Rohren (2) ausgerüstet ist, die zu einer Membranfiltereinheit (M) zum Aufkonzentrieren der Bakteriensuspension im Tank (T) durch tangentiale oder Cross Flow-Filtration führen, wobei der Tank (T) außerdem mit einer Rückführschleife (5) zur Zirkulierung des Tankinhaltes ausgerüstet ist und wobei der Tank (T) außerdem mit einer Austrittsleitung (6) zum Ausleiten des behandelten Inhalts des Tanks (T) ausgerüstet ist.

10. Verwendung einer Apparatur nach Anspruch 9 zur Herstellung eines Vakzins aus Vesikeln der proteinhaltigen äußeren Membran gegen eine Erkrankung, verursacht durch gramnegative Bakterien.

11. Verwendung nach Anspruch 10, wobei die gramnegativen Bakterien *Neisseria meningitidis* Serogruppe A sind.

## Revendications

1. Vaccin contre une bactérie méningococcique de sérogroupe A, **caractérisé en ce qu'**il comprend des vésicules protéiniques de membrane externe de la bactérie, les constituants protéiques étant présents principalement dans une conformation naturelle, éventuellement ensemble avec des véhicules et/ou des adjuvants acceptables pharmaceutiquement.

2. Vaccin suivant la revendication 1, **caractérisé en ce que** les protéines de la membrane externe comprennent les protéines Por B (P3.4 et P3.21) et Por A.

3. Vaccin suivant la revendication 2, **caractérisé en ce que** les protéines Por A sont P1.9 et P1.20.

4. Vaccin suivant la revendication 2 ou la revendication 3, **caractérisé en ce que** les protéines de membrane externe comprennent, en outre, Opc.

5. Procédé de production du vaccin suivant la revendication 1, dans lequel on cultive la bactérie méningococcique dans un fermenteur, puis on fait suivre d'une extraction des vésicules de membrane par du désoxycholate, de manière à former les vésicules de membrane externe.

6. Procédé de production du vaccin suivant la revendication 1, dans lequel on cultive la bactérie méningococcique dans un fermenteur, puis on fait suivre d'une extraction des vésicules de membrane par un détergent à base de sel d'acide biliaire, de manière à former des vésicules de membrane externe, le procédé étant effectué dans un système intégré fermé comprenant un fermenteur et une cuve de traitement à usage multiple communiquant avec un module de filtration à membrane.

7. Procédé suivant la revendication 6, qui comprend les stades dans lesquels :
a) on cultive les cellules de la bactérie dans le fermenteur ;
b) on transvase la suspension de cellules dans la cuve à buts multiples ;
c) on concentre la suspension de cellules en boucle à partir de la cuve à usages multiples par filtration tangentielle à courant croisé ;
d) on ajoute sous agitation un tampon à la cuve à usages multiples pour ajuster le pH à une valeur suffisamment haute pour ne pas précipiter le détergent à base de sel d'acide biliaire ;
e) on traite les cellules par le détergent à base de sel d'acide biliaire, de manière à inactiver la bactérie, à rompre la membrane externe et à former des vésicules de la membrane externe de la bactérie, les vésicules comprenant des constituants de membrane externe se présentant principalement sous leur forme naturelle ; et
f) on évacue l'extrait bactérien non infectieux contenant des vésicules de membrane externe.

8. Procédé suivant la revendication 7, dans lequel en outre :
a) on enrichit l'extrait bactérien obtenu en séparant les vésicules de membrane externe de cellules traitées et de débris ;
b) on effectue une centrifugation à grande vitesse du surnageant du stade (a) et on recueille les vésicules de membrane externe en une pastille ;
c) on disperse la pastille du stade (b) dans un tampon en agitant à température ambiante ;
d) on effectue une deuxième centrifugation à grande vitesse suivant le stade (b) et on recueille les vésicules de membrane externe en une pastille ;
e) on redisperse la pastille du stade (d) dans un milieu aqueux contenant un agent stabilisant en agitant à la température ambiante ;
f) on effectue une filtration pas à pas dans au moins deux filtres de dimension de pore décroissante de la composition redispersée provenant du stade (e) en terminant avec une dimension de pore du filtre d'environ 0,2 mm ; et
g) on incorpore éventuellement la composition provenant du stade (f) dans une composition de véhicule et/ou d'adjuvant acceptable pharmaceutiquement.

9. Installation propre à produire des vésicules de membrane externe à partir d'un milieu contenant une bactérie ne prenant pas le Gram, dans lequel le dispositif comprend un fermenteur (F) pour cultiver la bactérie en vue de former une suspension bactérienne, le fermenteur (F) communiquant par un conduit/canalisation (1) avec une cuve (T) à usages multiples, la cuve (T) communiquant par des conduits/canalisations (3, 4) avec un certain nombre de réservoirs (R, RI) auxiliaires contenant des liquides pour traiter la suspension bactérienne introduite dans la cuve (T) et dans laquelle la cuve (T) est équipée, en outre, de conduits/canalisations (2) menant à une unité (M) de filtres à membrane pour concentrer la suspension bactérienne dans la cuve (T) par filtration tangentielle ou à courant croisé, la cuve (T) étant équipée, en outre, d'une boucle (5) de réintroduction pour faire circuler le contenu de la cuve et la cuve (T) étant équipée, en outre, d'un conduit (6) de sortie pour évacuer le contenu traité de la cuve (T).

10. Utilisation de l'installation de la revendication 9 dans la préparation d'un vaccin protéinique à vésicules de membrane externe contre une maladie provoquée par une bactérie ne prenant pas le Gram.

11. Utilisation suivant la revendication 10, dans laquelle la bactérie qui ne prend pas le Gram est *Neisseria meningitidis* serogroupe A.
